# EUROPEAN PATENT APPLICATION

(11) **EP 2 896 702 A1**
(43) Date of publication of application: **22.07.2015**
(21) Application number: 14151458.8
(22) Date of filing: 16.01.2014
(51) Int. Cl.: C12Q 1/68

(54) **A method of identifying a neonate at risk of having or developing hypoxic-ischaemic encephalopathy (HIE)**

(71) Applicant: University College Cork, National University of Ireland Cork, Cork City (IE)
(72) Inventor: The designation of the inventor has not yet been filed
(74) Representative: Purdy, Hugh Barry

(57) **Abstract**

A method for screening a distressed neonate for risk of having or developing HIE comprises the steps of assaying a biological sample obtained from the distressed neonate, the mother of the neonate, or from the umbilical cord or placenta, for an abundance of miR-374a in the sample, and comparing the abundance of miR-374a in the sample with a reference abundance of miR-374a, wherein a reduced abundance of miR-374a in the sample compared with the reference abundance of miR-374a is indicative of the distressed neonate being at risk of having or developing HIE.

## Description

### Introduction

Neonatal hypoxic-ischaemic encephalopathy (HIE) describes the brain insult which results from insufficient oxygen or blood supply to the newborn brain during labour and delivery. HIE remains one of the leading causes of neurological disability worldwide. One in 50 newborn babies show signs of distress due to perinatal asphyxia (PA) following delivery. They may not cry, or breathe at birth, and will require immediate resuscitation. Of these 20% (4 per 1000) will go on to have hypoxic-ischaemic encephalopathy (HIE), with brain swelling, coma and seizures. In moderate and severe grades of HIE, over 50% of infants will be left with lasting brain injury, leading to cerebral palsy, learning difficulties, autism, epilepsy, visual or hearing impairment. This risk can be reduced significantly if infants are identified early and treated with induced hypothermia. This therapy has been shown to significantly improve the infant's chance of a normal outcome. However, to be beneficial it must be commenced within 6 hours of birth.

The problem to the clinicians looking after these infants is that we cannot accurately identify those babies who will benefit from hypothermia. Current blood markers (pH and lactate levels) are unreliable, but are measured in almost all infants who require resuscitation for fetal distress. EEG is currently our best method of predicting outcome. However this is difficult to record and interpret. It is also not currently available in a format which can be used in the labour ward. An early, reliable, quantifiable blood biomarker is badly needed.

It is an object of the invention to overcome at least one of the above-referenced problems.

### Statements of Invention

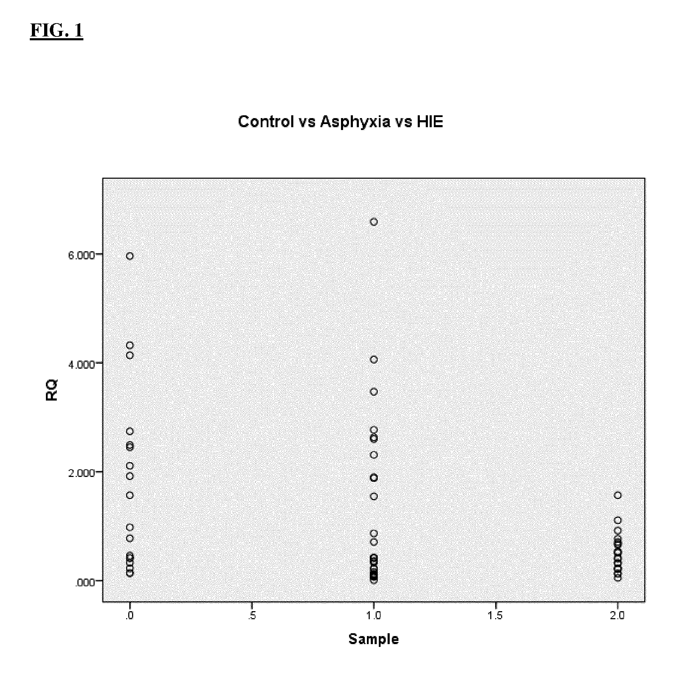

The Applicant has discovered a micro RNA molecule, micro RNA 374a (hereafter "miR-374a"), that is expressed in reduced abundance in a neonate that has or is at risk of developing severe or moderate HIE compared with a normal healthy neonate. This is shown in Fig. 1 and Table 1. In the cohorts tested, the mean RQ value for the control infants was 1.755, whereas the mean RQ values for the HIE infants was 0.5215 and for the Asphyxia infants was 1.10219. Thus, miR-374a levels in neonate or maternal blood can be used to differentiate infants with HIE (including patients with perinatal asphyxia) from normal infants, and distinguish moderate or severe HIE from normal infants. In particular, the invention may be employed to screen distressed neonates for risk of having or developing HIE, particularly moderate or severe HIE.

Accordingly, the invention provides a method of identifying risk of HIE in a neonate, typically a distressed neonate, comprising a step of assaying a biological sample obtained from the neonate, mother of the neonate, or placenta or umbilical cord for an abundance of miR-374a in the sample, wherein a reduced abundance of miR-374a in the sample relative to a reference abundance of miR-374a is indicative of a risk of HIE in the neonate.

In one embodiment, the invention provides a method of identifying risk of HIE in a distressed neonate comprising a step of assaying a biological sample obtained from the distressed neonate, mother of the distressed neonate, or placenta or umbilical cord within 6 hours of birth for an RQ-value of miR-374a in the sample, wherein an RQ-value of less than 1.1 is indicative of a risk of HIE in the neonate.

In another embodiment, the invention provides a method of identifying risk of moderate or severe HIE in a distressed neonate comprising a step of assaying a biological sample obtained from the distressed neonate, mother of the distressed neonate, or placenta or umbilical cord within 6 hours of birth for an RQ-value of miR-374a in the sample, wherein an RQ-value of less than 0.7 is indicative of a risk of moderate or severe HIE in the neonate.

In a further aspect, the invention provides a method of identifying risk of perinatal asphyxia in a neonate, comprising a step of assaying a biological sample obtained from the neonate, mother of the neonate, or placenta or umbilical cord prior to delivery for an abundance of miR-374a in the sample, wherein a reduced abundance of miR-374a in the sample relative to a pre-natal reference abundance of miR-374a is indicative of a risk of perinatal asphyxia in the neonate.

The invention provides a method of treating a neonate having or at risk of having HIE, the method comprising the steps of:
(a) identifying a neonate at risk of having HIE according to a method of the invention, and
(b) treating the neonate identified as being at risk of having HIE in step (a) with a neuroprotective therapy.

The invention also provides a system for determining whether a neonate is at risk of having or developing HIE, the system comprising:
(a) a determination module configured to receive at least one test sample (i.e. serum or whole blood) and perform at least one test analysis on the test sample to detect the abundance of miR-374a,
(b) optionally, a storage system for storing data relating to the abundance of the miR molecules generated by the determination system;
(c) a comparison module for comparing the detected abundance of the miR-374a with a reference abundance for the same micro RNA molecule
(d) a display module for displaying a content based in part on the data output from said determination module, wherein the content comprises a signal indicative of the presence or absence of decreased abundance of miR-374a relative to the reference abundance.

### Definitions

In this specification, the term "hypoxic-ischaemic encephalopathy" or "HIE" describes the brain insult which results from insufficient oxygen or blood supply to the newborn brain during labour and delivery, and consequent brain pathology, brain swelling or later brain injury. As employed herein, the term HIE should be understood to encompass mild, moderate or severe HIE. Moderate and severe HIE are characterised by lethargy, hypotonia, diminished deep tendon reflexes, occasional periods of apnoea, seizures, and absence of grasping, Moro, and sucking reflexes.

In most cases, the invention is directed to a method of screening a distressed neonate for risk of having or developing HIE, especially moderate or severe HIE. In this context, the term "distressed neonate" should be understood to mean a neonate that exhibits signs of fetal distress and requires resuscitation at birth. However, in certain embodiments, the invention may be employed to predict perinatal asphyxia, in which case the biological sample is assayed for miR-374 abundance, wherein detection of a reduced abundance of miR-374a in the sample relative to a healthy control reference abundance of miR-374a is indicative of a risk of perinatal asphyxia in the neonate. In certain embodiments, the invention may be employed to predict perinatal asphyxia prior to delivery, in which case the biological sample is taken during labor (for example, a sample of maternal blood).

In this specification, the term "micro RNA-374a" or "miR-374a" should be understood to mean the micro RNA described in the miRBase database under Accession number MI0000782 (WWW.MIRBASE.ORG). The sequence of miR-374a (mature), and miR374a (stem loop), are provided below as SEQUENCE ID NO'S 1 and 2, respectively.

### UUAUAAUACAACCUGAUAAGUG (SEQUENCE ID NO: 1)

### UACAUCGGCCAUUAUAAUACAACCUGAUAAGUGUUAUAGCACUUAUCAGAUU GUAUUGUAAUUGUCUGUGUA (SEQUENCE ID NO: 2)

In this specification, the term "neonate" should be understood to mean an infant mammal, typically an infant human, in the first 28 days after birth.

The term "at risk of having or developing HIE" refers to a risk that is greater than the 4 per 1000 risk that every neonate has of developing HIE following delivery. When the neonate being assessed is one that demonstrates signs of distress following delivery, the term "at risk of having or developing HIE" should be understood to mean a risk that is greater than the 1 per 5 risk that distressed neonates have of developing HIE following delivery.

In this specification, the biological sample from the neonate or mother should be understood to mean any biological sample including biological fluids such as blood, or a sample of cells or tissue, such as a sample of skin cells from the neonates scalp. The biological sample from the placenta or umbilical cord may be tissue, cells or blood, and the blood from the placenta or cord may be of venous or arterial origin. The term "blood" as used herein should be understood to mean blood or a blood derivative, for example plasma or serum. Generally, the biological sample is obtained postnatally, although in some circumstances it may be obtained prenatally, for example within 90 or 60 minutes before delivery. In most cases, the sample will be obtained post-natally, and generally within 6, 5, 4, 3, 2 or 1 hours of birth.

In this specification, the term "assaying the sample for an abundance of miR-374a in the sample" should be understood to mean determining the abundance of the micro RNA in either an absolute or relative manner. For example, in one embodiment of the invention, the abundance of miR-374a is determined is a relative manner using RQ-PCR. In this technique, the RQ stands for relative quantification and is generally measured as a delta-delta CT value. Essentially the abundance of a house keeping gene (for example, mir-223, however other housekeeping genes may be employed and will be known to those skilled in the art) and the target gene (mir-374a) is measured in the sample and the sample abundance is normalised according to that housekeeping gene. As the abundance of the housekeeping gene remains constant between all samples, the RQ value describes the change in expression of the target in relation to the housekeeping gene. In one embodiment, and using this technique, an RQ value of less that 1.1 is indicative of risk of HIE in the infant. The risk of HIE increases as the RQ value decreases, so that RQ values of less than 0.8 or 0.7 indicate a strong risk of HIE in the neonate, and in particular a strong risk of moderate or severe HIE.

In another embodiment, miR-374a can be determined in an absolute manner, using RT-PCR, and the absolute value correlated with risk by comparison with a reference value. Generally with this technique, the absolute value is normalised with a housekeeping gene to give a normalised mRNA copy number value, and it is this value that is compared with a normalised copy number value from a control sample. In one embodiment, the reference value from the healthy control may be a reference value from a pool of healthy controls, thus giving a mean healthy control value (or mean normalised healthy control value).

In this specification, the term "reduced abundance of miR-374a" should be understood to mean an abundance of miR-374a that is significantly reduced compared with a reference abundance of miR-374a.

The term "reference abundance of miR-374a" should be understood to mean an abundance of miR-374a in a distressed neonate that did not develop HIE - the reference value may be obtained from a single neonate, or more preferably is obtained from a cohort of neonates, so that the reference value represents a mean value for a sample of distressed neonates that did not develop HIE. When abundance of miR-374a is determined relatively using RQ values, typically an RQ value of less than 1.1 is indicative of risk of HIE, where the risk of HIE increases as the RQ value decreases (for example, RQ values of less than 1.0. 0.9, 0.8, 0.7 or 0.6). In one embodiment of the invention, an RQ value of less than 0.8 is indicative of risk of severe or moderate HIE, where the risk of severe or moderate HIE increases as the RQ value decreases (for example, RQ values of less than 0.6, 0.5 or 0.4).

In embodiments of the invention directed to screening for risk of perinatal asphyxia (either preor post-delivery), the reference level of miR-374a (described herein as "healthy control reference abundance of miR-374a") should be understood to mean an abundance of miR-374a in one or more healthy neonates exhibiting no signs of distress of perinatal asphyxia.

In this specification, the term "neuroprotective therapy" should be understood to mean treatment with therapeutic hypothermia, Xenon gas inhalation, stem cell transplantation, mast cell stabilisers, allopurinol, melatonin or any other neuroprotective agents or therapies.

The methods and systems for detecting risk of HIE as described herein are based on measuring the abundance of miR-374a in a distressed neonate and comparing the measured level with a reference level in one or more distressed neonates that did not develop HIE, in which reduced abundance relative to the reference level is indicative of risk of HIE. However, it will be appreciated that the reference level may be from a neonate with HIE, in which case a measured level that is similar to the reference level will indicate a risk of HIE.

The invention also provides a system for obtaining data from at least one test sample obtained from at least one individual, the system comprising:
(a) a determination module configured to receive at least one test sample (i.e. serum or whole blood) and perform at least one test analysis on the test sample to detect decreased abundance of miR-374a,
(b) optionally, a storage system for storing data relating to the abundance of the miR molecules generated by the determination system; and
(c) a display module for displaying a content based in part on the data output from said determination module, wherein the content comprises a signal indicative of the presence or absence of decreased abundance of miR-374a.

Preferably, the determination system comprises means for measuring the level of a micro RNA molecule (in absolute or relative terms) and then comparing the measured level with a reference level. The reference level may be a level of the same micro RNA from one or more healthy neonates, typically from the same type of sample.

Ideally, the determination system comprises a PCR apparatus.

The invention also provides a system for determining whether a neonate is at risk of having or developing HIE, the system comprising:
(e) a determination module configured to receive at least one test sample (i.e. serum or whole blood) and perform at least one test analysis on the test sample to detect the abundance of miR-374a,
(f) optionally, a storage system for storing data relating to the abundance of the miR molecules generated by the determination system;
(g) a comparison module for comparing the detected abundance of the miR-374a with a reference abundance for the same micro RNA molecule
(h) a display module for displaying a content based in part on the data output from said determination module, wherein the content comprises a signal indicative of the presence or absence of decreased abundance of miR-374a relative to the reference abundance.

In another embodiment, the system is for determining a suitable treatment for a neonate with HIE. Suitably, the comparison module is adapted to detect decreased abundance of miR-374a, and the display module is adapted to displaying a content based in part on the data output from said determination module, wherein the content comprises (a) a signal indicative of decreased abundance of miR-374a and/or (b) a content comprising a signal indicative of whether the individual is suitable for treatment with a neuroprotective therapy.

Embodiments of the invention also provide for systems (and computer readable media for causing computer systems) to perform a method for identifying the risk of a neonate, especially a distressed neonate, having HIE, or determining a suitable treatment for a neonate identified as being at risk of having HIE.

Embodiments of the invention can be described through functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions.

The functional modules of certain embodiments of the invention include at minimum a determination module, a storage module, optionally, a comparison module, and a display module. The functional modules can be executed on one, or multiple, computers, or by using one, or multiple, computer networks. The determination system has computer executable instructions to provide e.g., sequence information in computer readable form.

The storage module which can be any available tangible media that can be accessed by a computer. The storage module (i.e.computer readable storage media) includes volatile and nonvolatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (erasable programmable read only memory), EEPROM (electrically erasable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable storage media may define instructions, for example, as part of one or more programs, that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable storage media on which such instructions are embodied may reside on one or more of the components of either of a system, or a computer readable storage medium described herein, may be distributed across one or more of such components.

The computer-readable storage media may be transportable such that the instructions stored thereon can be loaded onto any computer resource to implement the aspects of the present invention discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects of the present invention. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The determination module can comprise any system for detecting increased or decreased abundance of the relevant micro RNA molecule. Standard procedures such as quantitative PCR can be used. Additionally one can determine other factors relevant to diagnosis of HIE, for example blood pH. These factors can be used in conjunction with levels of the relevant micro RNA molecule..The information determined in the determination system can be read by the storage device. As used herein the "storage device" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device is adapted or configured for having recorded thereon nucleic acid sequence information. Such information may be provided in digital form that can be transmitted and read electronically, e.g., via the Internet, on diskette, via USB (universal serial bus) or via any other suitable mode of communication.

As used herein, "stored" refers to a process for encoding information on the storage device. Those skilled in the art can readily adopt any of the presently known methods for recording information on known media to generate manufactures comprising information relating to micro RNA levels.

In one embodiment the reference data stored in the storage device to be read by the comparison module is compared for the purpose of detecting increased or decreased abundance of one or more specific micro RNA molecules compared with a reference abundance.

The "comparison module" can use a variety of available software programs and formats for the comparison operative to compare micro RNA abundance determined in the determination system to reference samples and/or stored reference data. In one embodiment, the comparison module is configured to use pattern recognition techniques to compare information from one or more entries to one or more reference data patterns. The comparison module may be configured using existing commercially-available or freely-available software for comparing patterns, and may be optimized for particular data comparisons that are conducted. The comparison module provides computer readable information related to the abundance of one or more micro RNA molecules in a sample relative to a reference abundance (i.e. relative to abundance of miR-374a in one or more healthy neonates).

The comparison module, or any other module of the invention, may include an operating system (e.g., UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (e.g., the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module provides a computer readable comparison result that can be processed in computer readable form by predefined criteria, or criteria defined by a user, to provide a content based in part on the comparison result that may be stored and output as requested by a user using a display module.

In one embodiment of the invention, the content based on the comparison result is displayed on a computer monitor. In one embodiment of the invention, the content based on the comparison result is displayed through printable media. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

In one embodiment, a World Wide Web browser is used for providing a user interface for display of the content based on the comparison result. It should be understood that other modules of the invention can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

The methods described herein therefore provide for systems (and computer readable media for causing computer systems) to perform methods as described above, for example (a) methods of detecting risk of a neonate, typically a distressed neonate, having HIE.

### Brief Description of the Figures

**Fig.** 1: Scatter plot of RQ values of miR-374a for control infants (0.0) vs asphyxia infants (1.0) vs HIE infants (2.0)

### Detailed Description of the Invention

### Material and Methods

### Patient Selection

Ethical approval for this study was obtained from the Clinical Research Ethics committee of the Cork Teaching Hospitals. The study was conducted from May 2009 to June 2011 in a single maternity hospital with 9000 deliveries per annum. Infants were identified as being at risk for HIE if they were over 36 weeks gestation with one or more of these previously published risk factors: an arterial cord pH <7.1, 5 minute Apgar score ≤6, or resuscitation at delivery required intubation. Parents of neonates meeting inclusion criteria were approached and written informed consent obtained. After enrolment clinical and demographic details on all infants were recorded prospectively. Grade of encephalopathy was assigned at 24 hours of life by a dedicated research fellow, using the modified Sarnat score. Standardised neurological assessment was additionally performed on day 3 and at discharge.

Case infants were divided into those with HIE, and those with biochemical or clinical risk of asphyxia without clinical encephalopathy (Asphyxia) based upon this examination.

A control population was recruited over the same period as part of an ongoing birth cohort study (The BASELINE Study www.baselinestudy.net). Ante-natal parental consent was obtained for all control infants enrolled. The control population were all full term infants, born by unassisted vaginal delivery, without any medical issues. All had normal examinations, were not admitted to the neonatal unit, and did not have EEG monitoring.

All case infants had continuous multi-channel EEG recorded, commencing in the first 24 hours of life. The background EEG was graded according to a modification of a standardized HIE grading system. The entire recording was reviewed for the presence of electrographic seizures, which were defined as stereotyped repetitive discharges on one or more channels, with a clear evolution, that lasted for > 10 seconds.

### MiRNA analysis

### Sample collection, and RNA extraction

Umbilical cord blood was drawn on all infants. 3ml of cord blood was placed into TempusTM Blood RNA tubes (Applied Biosystems, Foster City, CA). The tubes were then agitated for 10 seconds to ensure that the reagent made uniform contact with the sample, before being biobanked at -80 C. Once sufficient samples were collected, the RNA was extracted from the Tempus system using the MagMAX™ for Stabilized Blood Tubes RNA Isolation Kit (Applied Biosystems/Ambion, Austin, Tx). The concentration of the RNA was determined using a NanoDrop Spectrophotometer (Rockland, DE).

### MiRNA microarray assay

For the microarray assay a commercial provider was used (Beckman Coulter Genomics Inc., Fullerton, Ca.). Beckman Coulter use the Agilent Human miRNA Microarray Version 3.0 (Agilent technologies Inc., Agilent Laboratories, Santa Clara, Ca.). This system contains probes for 866 human miRNA from the Sanger miRBase 12.0 Release (http://www.mirbase.org). In brief the system directly labels the miRNA, adding a single 3' Cytidine and one cyanine dye to the 3' end. When developing their probes, Agilent added a Guanine base to the 5' end of each, this complements the 3' Cytidine added during labeling, allowing G-C pair to form, stabilising the targeted miRNA. This stabilisation allows equalisation of the melting temperatures of the probe-target hybrids. Additionally there is a further 5' hairpin on the probes, which increases the target specificity of the probe, preventing stable hybridization of longer non-target miRNAs.

### MiRNA real time PCR

Quantitative reverse transcription polymerase chain reaction (RT-PCR) was performed for hsa-mir-374a, to validate the microarray results. For this analysis the miRCURY LNA™ Universal RT microRNA PCR (Exiqon, Woburn, Ma) was used with pre designed primers (Exiqon, Woburn, Ma) for the miRNA of interest (hsa-mir-374a} and housekeeper miRNA (has-mir-223).

All analysis was performed as per the manufacturer's protocols for individual assays using whole blood samples. In brief, RT master mix was made for each of the primers as per the kits protocols. First-strand cDNA synthesis was then performed by adding 16 µL of RT master mix to 4 µL of template total RNA. This was incubated at 42°C for 60 min, and then inactivated by heating to 95 °C for 5 min. The cDNA was then added to the PCR master mix (PCR primer and SYBR Green master mix) and centrifuged at 1500g for 1 min to ensure all reagents were mixed. For amplification all reactions were performed in duplicate, at a final volume of 10 µL per well, using Rotor Gene 6000. Polymerase activation and denaturation was performed at 95°C for 10 min, followed by 40 amplification cycles of 95°C for 10 s and 60°C for 60 s, with a ramp-rate of 1.6 °C/s. At the end of the PCR cycles, melting curve analyses were performed. Threshold values for threshold cycle determination (Ct) were generated for each of the duplicate amplification reactions, and the mean calculated. The miRNA fold change relative to the housekeeper miRNA was then calculated using the delta-delta Ct method.

The miR-374a levels for all patients are provided below in Table 1.
RQ

**Table 1: Description statistics of all sample groups (Control, Asphyxia, HIE)**

| | N | Mean | Std. Deviation | Std. Error | 95% Confidence Interval for Mean | | Minimum | Maximum |
|---|---|---|---|---|---|---|---|---|
| | | | | | Lower Bound | Upper Bound | | |
| Control | 18 | 1.75500 | 1.689460 | .398209 | .91485 | 2.59515 | .140 | 5.960 |
| Asphyxia | 32 | 1.10219 | 1.520833 | .268848 | .55387 | 1.65051 | .010 | 6.590 |
| HIE | 20 | .52150 | .373508 | .083519 | .34669 | .69631 | .050 | 1.570 |
| Total | 70 | 1.10414 | 1.410565 | .168595 | .76781 | 1.44048 | .010 | 6.590 |

The invention is not limited to the embodiments hereinbefore described which may varied in construction and detail without departing from the spirit of the invention.

## Claims

1. A method of identifying risk of HIE in a distressed neonate, comprising a step of assaying a biological sample obtained from the distressed neonate, mother of the neonate, or placenta or umbilical cord, for an abundance of miR-374a in the sample, wherein a reduced abundance of miR-374a in the sample relative to a reference abundance of miR-374a is indicative of a risk of HIE in the distressed neonate.

2. A method as claimed in Claim 1 in which the biological sample is blood obtained from the umbilical cord or placenta.

3. A method as claimed in Claim 1 or 2 in which the biological sample is obtained within six hours after delivery.

4. A method as claimed in any preceding Claim comprising a step of assaying a biological sample obtained from the neonate, the mother of the neonate, or from the umbilical cord or placenta, within 6 hours of birth for an RQ-value of miR-374a in the sample, wherein an RQ-value of less than 1.1 is indicative of a risk of HIE in the distressed neonate.

5. A method as claimed in any preceding Claim comprising a step of assaying a biological sample obtained from the neonate, the mother of the neonate, or from the umbilical cord or placenta, within 6 hours of birth for an RQ-value of miR-374a in the sample, wherein an RQ-value of less than 0.7 is indicative of a risk of moderate or severe HIE in the distressed neonate.

6. A method of treating a neonate having or at risk of having HIE, the method comprising the steps of:
(a) identifying a neonate at risk of having HIE according to a method of any of Claims 1 to 6, and
(b) treating the neonate identified as being at risk of having HIE in step (a) with a neuroprotective therapy.

7. A method as claimed in Claim 7, in which steps (a) and (b) are carried out within 6 hours of delivery of the neonate.

8. A method as claimed in Claim 7 or 8 in which the neuroprotective therary is induced hypothermia.

9. A method of identifying a neonate suitable for treatment with induced hypothermia for treatment of HIE, the method comprising the step of (a) identifying a neonate at risk of having or developing HIE according to a method of any of Claims 1 to 5, and (b) treating the neonate identified as being at risk of having or developing HIE in step (a) with induced hypothermia.

10. A method as claimed in Claim 9, in which steps (a) and (b) are carried out within 6 hours of delivery of the neonate.

11. A system for determining whether a neonate is at risk of having or developing HIE, the system comprising:
(a) a determination module configured to receive at least one test sample comprising or derived from neonate or maternal blood and perform at least one test analysis on the test sample to detect the abundance of miR-374a,
(b) optionally, a storage system for storing data relating to the abundance of the miR-374a generated by the determination system;
(c) a comparison module for comparing the detected abundance of the miR-374a with a reference abundance for the same micro RNA molecule from one or more healthy neonates; and
(d) a display module for displaying a content based in part on the data output from said determination module, wherein the content comprises a signal indicative of risk of HIE when the detected abundance of miR-374a is reduced relative to the reference abundance.

12. A method of identifying risk of perinatal asphyxia in a neonate, comprising a step of assaying a biological sample obtained from the mother, foetus, or placenta or umbilical cord prior to delivery for an abundance of miR-374a in the sample, wherein a reduced abundance of miR-374a in the sample relative to a healthy control reference abundance of miR-374a is indicative of a risk of perinatal asphyxia.

13. A method as claimed in Claim 12 in which the biological sample is obtained during labor.

14. A method as claimed in Claim 12 or 13 in which an RQ-value of miR-374a in the biological sample obtained, wherein an RQ-value of less than 1.7 is indicative of a risk of perinatal asphyxia in the neonate.


SEQUENCE LISTING

<110> UNIVERSITY COLLEGE CORK, NATIONAL UNIVERSITY OF IRELAND,
CORK

<120> A method of identifying a neonate at risk of having or developing
hypoxic-ischaemic encephalopathy (HIE)

<130> P11353EP00

<160> 2

<170> PatentIn version 3.5

<210> 1
<211> 22
<212> RNA
<213> Homo sapiens

<220>
<221> misc_RNA
<222> (1)..(22)
<223> hsa-miR-374a mature sequence

<400> 1
uuauaauaca accugauaag ug 22

<210> 2
<211> 72
<212> RNA
<213> Homo sapiens

<220>
<221> misc_RNA
<222> (1)..(72)
<223> hsa-miR-374a stem loop

<400> 2
uacaucggcc auuauaauac aaccugauaa guguuauagc acuuaucaga uuguauugua 60

auugucugug ua 72